# EUROPEAN PATENT SPECIFICATION

(11) **EP 1 985 252 B1**
(45) Date of publication and mention of the grant of the patent: **09.03.2011**
(21) Application number: 08011259.2
(22) Date of filing: 24.05.2006
(51) Int. Cl.: A61B 18/14

(54) **High frequency treatment tool and mucous membrane exfoliation method using the high frequency treatment tool**
Hochfrequenzbehandlungswerkzeug und Mukosemembran-Abtragungsverfahren mit dem Hochfrequenzbehandlungswerkzeug
Outil de traitement à haute fréquence et procédé d'exfoliation de membrane muqueuse utilisant l'outil de traitement à haute fréquence

(30) Priority: 25.05.2005 JP 2005151795
(43) Date of publication of application: 29.10.2008
(62) Divisional of application: 06010716.6
(73) Proprietor: FUJIFILM Corporation, Minato-ku Tokyo (JP); Toyonaga, Takashi, Izumi-shi Osaka (JP)
(72) Inventor: Machiya, Mamoru c/o Fujinon Corporation, Saitama (JP); Ooyatsu, Masayuki c/o Fujinon Corporation, Saitama (JP); Toyonaga, Takashi, Osaka (JP)
(74) Representative: Klunker . Schmitt-Nilson . Hirsch

(56) References cited:
- US-A- 4 708 137
- US-A1- 2004 210 284
- PATENT ABSTRACTS OF JAPAN vol. 2003, no. 12, 5 December 2003 (2003-12-05) & JP 2005 270241 A (PENTAX CORP), 6 October 2005 (2005-10-06)

## Description

### Background of the Invention

### 1. Filed of the Invention

The invention relates to a high frequency treatment tool that can be used for treatment such as removal of a diseased mucous membrane by incising and exfoliating it by being inserted in a treatment tool insertion channel of an endoscope, and can supply a biocompatible liquid such as normal saline solution, sodium hyaluronate, or glyceol.

### 2. Description of the Related Art

When a diseased portion such as a tumor is found on the mucous membrane on the body cavity inner wall of the gullet, the stomach, the duodenum, the colon, or the like by an endoscopic examination, treatment is performed to excise the diseased mucous membrane. One of such treatment is called endoscopic submucosal dissection (ESD). This ESD is normally carried out as follows. First, the portion of the mucous membrane to be excised is marked, and the portion of the diseased mucous membrane is bulged by means of local injection. In this state, the mucous membrane is incised along the marking by using a high frequency treatment tool and the fibers of the submucosal layer are cut and the mucous membrane is exfoliated from the muscle layer.

The high frequency treatment tool to be used for the above-described treatment is formed by attaching a high frequency knife including an electrode member having a bar-shaped portion inside a flexible sheath. To the base end of the flexible sheath, operating means is joined, and by this operating means, the high frequency knife is stuck out from the front end of the flexible sheath. By energizing the high frequency knife, the mucous membrane can be incised and exfoliated. As the knife of the high frequency treatment tool to be used for this ESD, there are available a needle-shaped knife including an electrode member extending straight, and a hook knife having a hook portion formed by providing a large-diameter electrode portion continuously from the front end of the bar-shaped electrode member or bending the front end into a roughly L shape. The needle-shaped knife is most suitable for piercing the mucous membrane, and the mucous membrane can be incised or exfoliated by horizontally moving and swinging the electrode member. On the other hand, the hook knife incises or exfoliates the mucous membrane by hooking the mucous membrane on the hook portion on the front end and drawing it. Such a hook knife is known for example from US 2004/210284.

The muscle layer is under the mucous membrane, and two carry out the treatment, the high frequency knife must be operated so as not to damage this muscle layer, that is, so as not to come into contact with the muscle layer when the high frequency knife is energized. Therefore, the high frequency knife, in particular, the front end of the high frequency knife must always be captured within the observation field of the endoscope. However, the needle-shaped knife is inserted into the mucous membrane, so that depending on the circumstances, the front end comes out of the observation field of the endoscope, so that it is difficult to perform treatment while completely preventing the front end of the needle-shaped knife from coming into contact with the muscle layer. On the other hand, in the case of using the hook knife, the hook knife is hooked on the mucous membrane under observation with the endoscope, drawn into the treatment tool insertion channel, andenergized to cut the tissue, whereby incising or exfoliating the mucous membrane. Therefore, the hook knife can be operated under observation with the endoscope, and it does not come into contact with the muscle layer in an energized state, so that the method using the hook knife is superior in terms of treatment safety.

JP-A-2004-313537 proposes a hook knife provided with a mechanism for more stably retaining the position of the front end of the hook knife when hooking. In the high frequency treatment tool of JP-A-2004-313537, an electrical insulating member is attached to the front end of the flexible sheath, and a through hole is made in the electrical insulating member, and the bar-shaped portion of the electrode member of the hook knife is inserted in this through hole, and the hook portion on the front end can come into contact with and separate from the front end outer surface of the electrical insulating member. When energized, the electrode member is stuck out a predetermined length from the flexible sheath, and the diameter difference between the diameter of the through hole and the diameter of the electrode member is minimized, and the sticking-out length of the electrode member is restricted, whereby the electrode member is stably retained. In the maximum sticking-out state of the electrode member, at least the hook portion is set so as to be captured in the observation field of the endoscope.

As described above, by stably retaining the portion of the electrode member stuck-out from the flexible sheath, the direction of the electrode member can be easily controlled, and this is advantageous for safe operation for hooking and cutting the tissue. However, the operation for hooking the mucous membrane and the submucosal layer by the hook knife of the electrode member and drawing the knife into the treatment tool insertion channel while energizing to cut the tissue and leading the hook knife out from the treatment tool insertion channel is repeatedlyperformed, so that the operation efficiency and swiftness are not obtained. Therefore, the treatment of removing the diseased mucous membrane takes a long time and accordingly increases the pain of the examinee being subjected to treatment and the burden on the operator. The hook portion is always exposed to the outside, and for example, during insertion into the treatment tool insertion channel, if the electrode member is energized by mistake, it may damage the channel inner wall.

### Summary of the Invention

The invention was developed in view of the above-described circumstances, and an object thereof is to provide a high frequency treatment tool with which treatment such as incision and exfoliation of the mucous membrane can be safely, swiftly, and efficiently performed.

In order to achieve the above-described object, a high frequency treatment tool to be inserted in a body cavity via a treatment tool insertion channel of an endoscope, comprises: a flexible sheath capable of being inserted in the treatment tool insertion channel; a treatment tool main body comprising a flexible cord and a straight electrode member that can apply a high frequency current on the front end of the flexible cord, the treatment tool main body being provided inside the flexible sheath; a stopper member comprising an electrical insulating material, the stopper member being attached inside the flexible sheath and disposing its front end face at almost the same position as a front end of the flexible sheath so as to form a front end reference face; an insertion hole that is formed so as to penetrate the stopper member in its axial direction, and in which the electrode member is to be inserted; an operating section that is connected to a base end of the flexible cord and reciprocates the electrode member between a state that the electrode member is stuck out from the front end reference face and a state that the electrode member is withdrawn into the insertion hole by pushing or pulling the treatment tool main body within the flexible sheath; a restricting member that is provided at or near a connecting portion between the flexible cord and the electrode member and can come into contact with and separate from a base end of the stopper member to restrict the maximum sticking-out length of the electrode member from the front end reference face; and a fluid channel that is provided at the stopper member and makes a fluid to flow out from the front end face.

The high frequency treatment tool is inserted in the treatment tool insertion channel of the endoscope, and treatment such as incision and exfoliation of the mucous membrane is performed, however, capturing of the front end of the electrode member provided at the front end of the treatment tool main body in the observation field of the endoscope is not essential for safety of the treatment. By adjusting the sticking-out length of the electrode member from the flexible sheath so as not to come into contact with the muscle layer when the front end of the electrode member penetrates the mucous membrane, the front end of the electrode member being unable to be recognized does not especially pose a problem as long as the front end of the flexible sheath is captured in the observation field of the endoscope.

Herein, the treatment of excision of the diseased mucous membrane is effective in the case where the surface of the mucous membrane is diseased and the diseased portion does not infiltrate into the submucosal layer. The submucosal layer is between the mucous membrane and the muscle layer. Therefore, when excising the mucous membrane, the entire region of the diseased mucous membrane must be removed.

By considering these points, the front end face of the flexible sheath is formed so as to come into contact with the mucous membrane surface, and the sticking-out length of the electrode member from the front end face of the flexible sheath is set so as not to reach the muscle layer although it penetrates the mucosal layer, whereby safe treatment is possible and the mucosal layer can be exfoliated without fail, and the muscle layer is not damaged. A restricting member provided on the treatment tool main body comes into contact with a stopper member attached to the front end of the flexible sheath to restrict the maximum sticking-out length of the electrode member. Therefore, the straight electrode member, that is, the electrode member having the shape of the needle-shaped knife can be used, and with this, by horizontally moving and swinging the electrode member without hooking, incision and exfoliation can be performed swiftly and efficiently. Herein, depending on the organ to be treated, the thicknesses of the mucous membrane and the submucosal layer are different. Therefore, it is desirable that a plurality of types of electrode members with different maximum sticking-out lengths are prepared according to the portion to be treated. By making the position of the stopper member or the restricting member adjustable, the maximum sticking-out length of the electrode member can be changed according to the portion to be treated, however, by considering danger in treatment in a state from failing to adjust, such an adjusting mechanism is not provided.

To stably retain the front end face of the flexible sheath in contact with the mucous membrane surface, the front end of the flexible sheath must have a wide face. The front end face of the stopper member is set as a front end reference face by facing it with the position of the front end of the flexible sheath, and thereby, a wide front end reference face is formed by the end face of the flexible sheath and the end face of the stopper member. As a result, when the front end reference face is made contact with the mucous membrane, the pressure per unit area can be reduced, so that the mucous membrane can be retained without great deformation. Even when a difference in level is formed between the front end of the flexible sheath and the front end of the stopper member, if the difference is slight, it does not functionally pose a problem.

The electrode member is straight, so that it can be stuck out from and withdrawn into the in sertion hole of the stopper member. The stopper member is made of an electrical insulating material, so that when the high frequency treatment tool is inserted in the treatment tool insertion channel, by drawing the electrode member to the position to the base end side through the insertion hole, the inner surface of the treatment tool insertion channel is prevented from being damaged even when the power source is actuated by mistake and the electrode member is energized. The stopper member can be made of, for example, plastic as long as it has electrical insulation, however, there is a possibility that the electrode member generates heat when the electrode member is stuck out from the stopper member and a high-frequency current is supplied, so that the stopper member is desirably made of ceramic in terms of heat resistance and shape retention.

When the treatment such as incision and exfoliation of the mucous member is not performed, the electrode member is retained in a state that it is not stuck out from the stopper member, however, if the flexible sheath is bent, the treatment tool main body moves axially inside. As a result, the front end of the electrode member may stick out from the front end face of the stopper member although this is not intended. To retain the electrode member in the non-sticking-out state without fail, the stopper member is lengthened and the insertion hole is lengthened, however, it is desirable that the electrode member is positioned more inwardly than the stopper member attached to the flexible sheath. To reliably guide the electrode member drawn-in from the stopper member into the insertion hole when it is stuck out, a draw-in tapered portion for drawing the electrode member into the insertion hole is provided on the base end face of the stopper member. By providing a center alignment function between the electrode member and the insertion hole, the electrode member can be guided into the insertion hole. For this, the difference of the outer diameter of the restricting member from the inner diameter of the flexible sheath is reduced, in detail, the clearance between the restricting member and the inner surface of the flexible sheath is set to be smaller than the clearance between the electrode member and the insertion hole, and some degree of length is secured axially. However, if the restricting member is made of a hard material, it becomes unbendable. By forming the restricting member of, for example, a close coil spring, it becomes bendable. Against the stopper, the restricting member is pressed, so that the stopper member is desirably firmly fixed to the flexible sheath so as not to come off the flexible sheath. The stopper member is fixed by means of bonding to the inner surface of the flexible sheath, however, to increase the fixing strength, for example, the base end side outer circumferential surface of the stopper member to be inserted in the flexible sheath is increased in diameter to form a stepped structure.

The high frequency treatment tool has a liquid supply means. To bulge the diseased mucous membrane, a biocompatible liquid such as normal saline solution, sodium hyaluronate, or glyceol is locally injected, and the same liquid as this locally injected liquid is supplied. Particularly, normal saline solution infiltrates into the body and flows out during operation, so that the bulging portion shrinks after a certain time elapses, and the bulging portion disappears soon. By considering this point, the liquid supply means is used as replenishing means for maintaining the bulging portion formed by local injection. In detail, for example, a pipe-shaped member is joined to the base end of the flexible sheath, and this joined pipe is provided with a liquid feed means connecting portion. The liquid is jetted from the front end reference face. The liquid can be efficiently replenished to a target portion of the submucosal layer by jetting it with a high pressure while making the front end reference face contact with the mucous membrane or the submucosal layer exposed by incising the mucous membrane. The stopper member is contacted with the restricting member, so that one or a plurality of grooves are provided on the outer circumferential surface of this stopper member so as to serve as liquid jetting paths. To prevent the jetting paths from being clogged by the restricting member, the outer diameter of the restricting member is made smaller than the outer diameter of the stopper member. Thereby, the grooves are reliably communicated with the passage inside the flexible sheath.

There is preferably provided the high frequency treatment tool described above, wherein a body cavity inner wall of the body cavity comprises a mucosal layer, a submucosal layer and a muscle layer in this order from a surface of the body cavity, and the maximum sticking-out length of the electrode member from the front end reference face is set equal to or more than a thickness of the mucous layer and equal to or less than a depth from a surface of the mucous layer to the muscle layer. Namely, the maximum sticking-out length is usually from 0.5 mm to 4 mm, and is preferably from 1 mm to 3 mm.

There is also preferably provided the high frequency treatment tool described above,
wherein a front end of the electrode member has a displacement stroke from: a maximum position at which the electrode member has the maximum sticking-out length; to a minimum position at which the front end of the electrode member is withdrawn from a base end of the stopper member to abase end side of the high frequency treatment tool.

There is also preferably provided the high frequency treatment tool described above, wherein on the base end face of the stopper member, a draw-in tapered portion is provided for drawing the electrode member in the insertion hole.

There is also preferablyprovidedthe high frequency treatment tool described above, wherein the stopper member comprises ceramic, a diameter of the insertion hole is set so that the electrode is inserted therein without substantial gaps, the flow channel is formed by one or a plurality of grooves formed on an outer circumferential surface of the stopper member, and an outer diameter of the restricting member is set smaller than an outer diameter of the stopper member.

### Brief Description of the Drawings

Fig. 1 is an entire construction view of the high frequency treatment tool showing an embodiment of the invention;
Fig. 2 is a main part enlarged sectional view of Fig. 1;
Fig. 3 is an enlarged sectional view of the front end portion of the treatment tool main body;
Fig. 4 is a sectional view similar to Fig. 3, showing a state that the electrode member is stuck out;
Fig. 5 is a sectional view on X-X of Fig. 4;
Fig. 6 is an external view showing a state that the high frequency treatment tool of an embodiment of the invention is led out from a treatment tool insertion channel of an endoscope;
Fig. 7 is a plan view showing a state that the diseased mucous membrane region is marked;
Fig. 8 is a sectional view of the tissue, showing local injection into the diseased mucous membrane region;
Fig. 9 is a sectional view of the tissue, showing incision by using the high frequency treatment tool;
Fig. 10 is a plan view of the diseased mucous membrane region, showing a state that incision with the high frequency treatment tool is finished;
Fig. 11 is a sectional view of the tissue, showing exfoliation of the mucous membrane;
Fig. 12 is a sectional view of the front end portion of the treatment tool main body of the second embodiment of the invention;
Fig. 13 is a sectional view of the front end portion of the treatment tool main body of the third embodiment of the invention; and
Fig. 14 is a sectional view of the front end portion of the treatment tool main body of the fourth embodiment of the invention;

### Detailed Description of the Invention

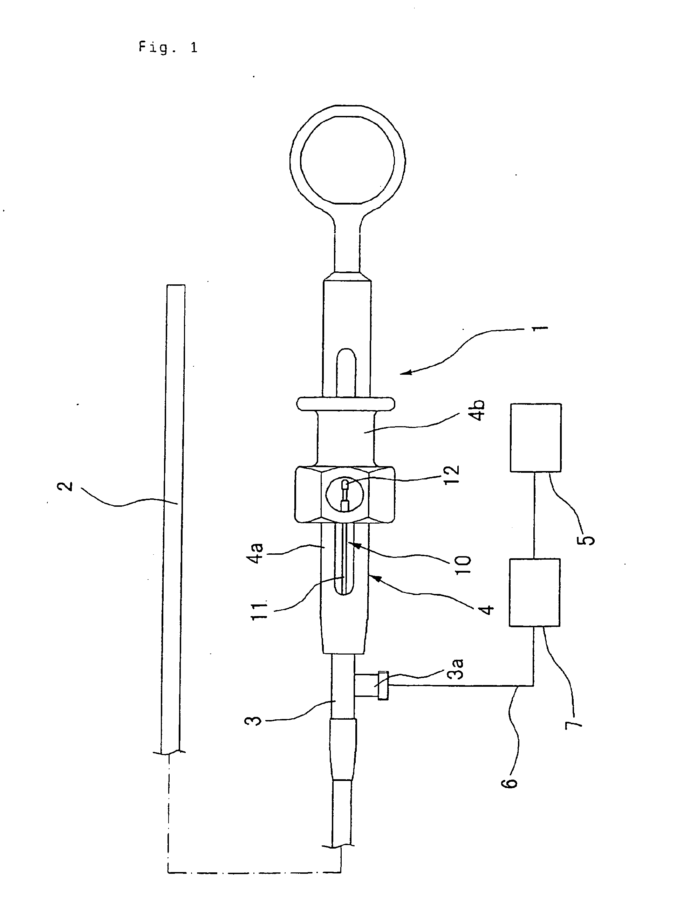

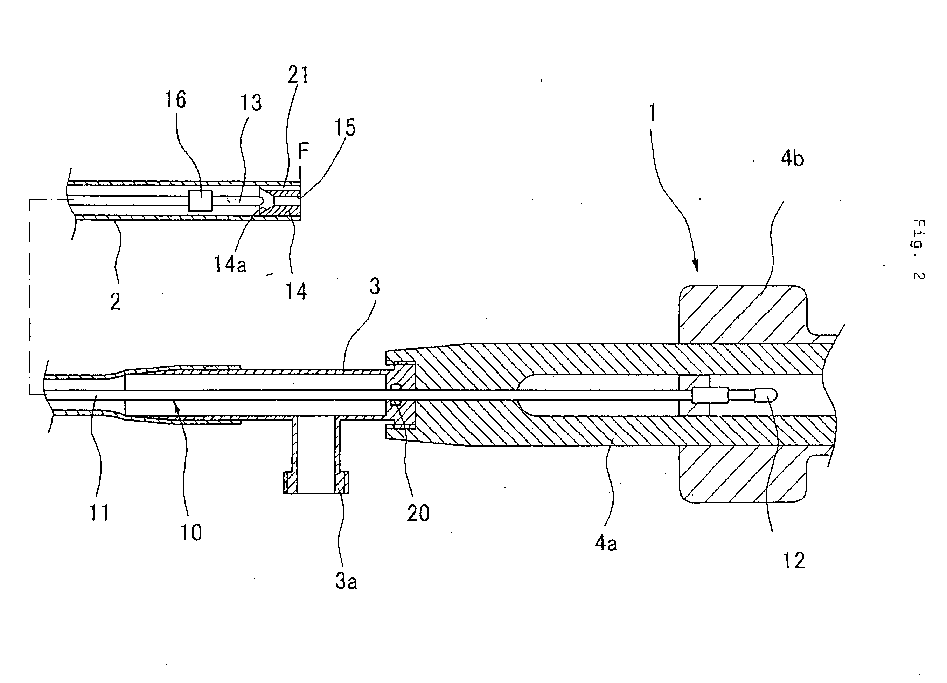

Hereinafter, embodiments of the invention will be explained with reference to the drawings. First, Fig. 1 shows an entire construction of a high frequency treatment tool, and Fig. 2 is a main part enlarged sectional view of the same. In the figures, the reference numeral 1 denotes a high frequency treatment tool, and this high frequency treatment tool 1 has a long flexible sheath 2, and a connecting pipe 3 is joined to the base end of this flexible sheath 2, and operating means 4 is joined to the other end of this connecting pipe 3. The operating means 4 includes a main body shaft 4a joined to the connecting pipe 3 and a slider 4b that is fitted to the main body shaft 4a and is slidable in the axial direction of the main body shaft 4a. To the slider 4b, the base end of the flexible cord 11 of the treatment tool main body 10 is joined. The flexible cord 11 is formed by coating an electrical insulating material such as a fluorine resin on the outer circumference of a lead wire, and the base end thereof sticks out by a predetermined length from the portion joined to the slider 4b and is provided with a contact portion 12. This contact portion 12 is disconnectably connected to a high frequency power source unit (not shown in the figure).

As clearly seen in Fig. 2, the flexible cord 11 of the treatment tool main body 10 is extended from the portion connected to the slider 4b to the inside of the flexible sheath 2 through the inside of the connecting pipe 3. A lead wire is extended straight from the front end of the flexible cord 11, and the led-out portion of this lead wire is formed into an electrode member 13 forming the needle-shaped knife. A stopper member 14 is inserted and fitted to the front end of this flexible sheath 2, and is fixed by means of bonding or the like. The stopper member 14 is made of ceramic, and the front end face thereof is disposed at the same position as the front end face of the flexible sheath 2, and therefore, the front end face of the stopper member 14 and the front end face of the flexible sheath 2 form a front end reference face F. At the position of the central axis line of the stopper member 14, an insertion hole 15 is formed to penetrate in the axial direction, and the hole diameter of this insertion hole 15 is set to be slightly larger than the outer diameter of the electrode member 13. On the base end of the stopper member 14, a draw-in tapered portion 14a is formed toward the insertion hole 15.

At the shift portion from the flexible cord 11 to the electrode member 13 in the treatment tool main body 10 or at the portion of the electrode member 13, a restricting member 16 is attached. The restrictingmember 16 is larger in diameter than at least the insertion hole 15, and therefore, when the treatment tool main body 10 is advanced inside the flexible sheath 2 and the electrode member 13 sticks out by a predetermined length from the front end reference face F, the restricting member 16 comes into contact with the stopper member 14 and restricts the electrode member 13 from sticking out more. That is, the maximum sticking-out position of the electrode member 13 is regulated.

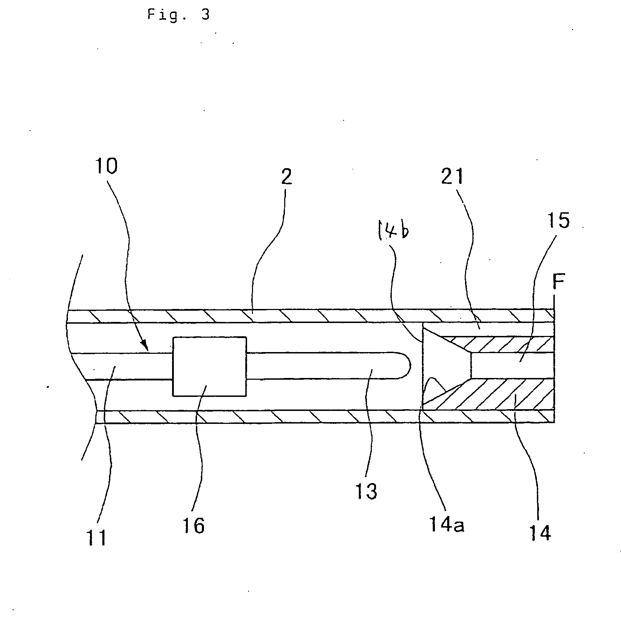

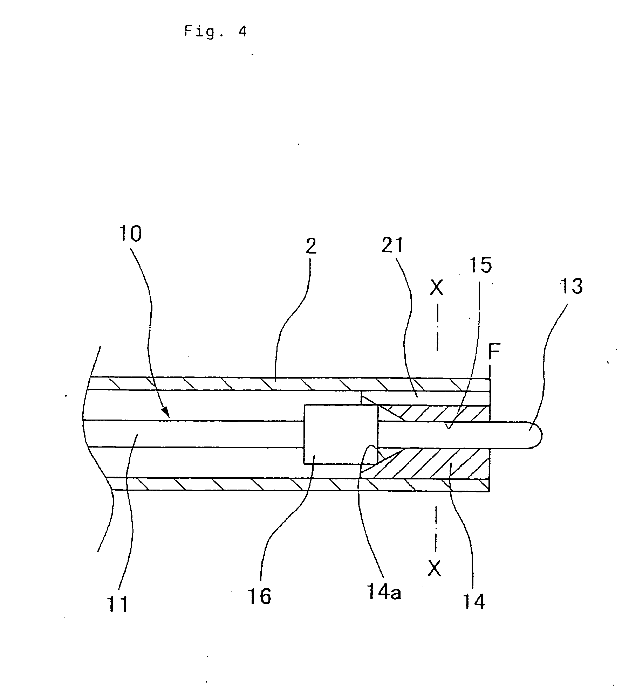

Fig. 3 shows a maximum drawn-in state of the electrode member 13, and Fig. 4 shows a maximum sticking-out state of the electrode member 13. In the maximum drawn-in state of the electrode member 13, the front end.of the electrode member 13 is withdrawn from a base end 14b of the stopper member 14 to a base end side of the high frequency treatment tool 1. The maximum sticking-out length of the electrode member 13 from the front end reference face F depends on the thickness of the mucosal layer to be treated. As described later, between the mucosal layer and the muscle layer, the submucosal layer is present. To incise and exfoliate the mucous membrane, the sticking-out length of the electrode member 13 is set to a length longer than the thickness of the mucosal so that the front end of the electrode member 13 does not reach the muscle layer when the front end reference face F is made contact with the mucous membrane surface. Thereby, when the electrode member 13 is stuck out to the maximum sticking-out state while the front end reference face F is in contact with the mucous membrane surface, this electrode member 13 reliably penetrates the mucosal layer and does not reach the muscle layer. The pushing and pulling of this electrode member 13 can be made by remote control from the operating means 4.

Furthermore, this high frequency treatment tool 1 has means for supplying a biocompatible liquid, for example, normal saline solution. This supply means has, as clearly seen in Fig. 1, a connection port 3a provided in the connecting pipe 3, and to this connection port 3a, a liquid feed pipe 6 from a water tank 5 is disconnectably connected. At an intermediate point of this liquid feed pipe 6, switching means 7 for opening and closing the flow channel like a foot switch, etc., is provided to control the supply of the normal saline solution. Therefore, the inside of the flexible sheath 2 joined to the connecting pipe 3 is used as a liquid feed channel. Herein, the flexible cord 11 of the treatment tool main body 10 is joined to the slider 4b of the operating means 4 via the connecting pipe 3 from the flexible sheath 2, and a seal member 20 is attached around the flexible cord 11 in the connecting pipe 3 to prevent backward flow of the normal saline solution.

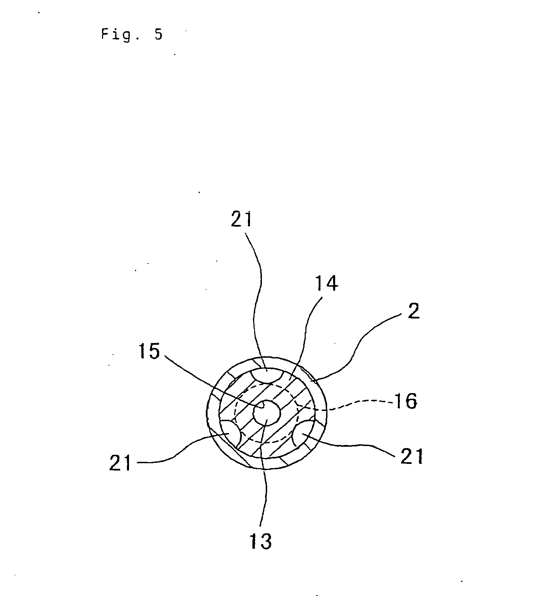

The normal saline solution can be jetted forward from the front end of the flexible sheath 2. Therefore, as shown in Fig. 5, on the outer circumferential surface of the stopper member 14 attached inside the flexible sheath 2, a plurality (three in the drawings) of grooves 21 are formed at equal intervals circumferentially. These grooves 21 have a length covering the entire length in the axial direction of the stopper member 14, and serve as normal saline solution jetting paths. Herein, when the electrode member 13 is stuck out, the restricting member 16 comes into contact with the stopper member 14, however, by setting the outer diameter of the restricting member 16 to be smaller than that of the stopper member 14, preferably, by setting the outer diameter to be almost the same as the diameter of the circle connecting the bottoms of the grooves 21, the jetting paths formed by the grooves 21 are secured even when the restricting member 16 comes into contact with the stopper member 14.

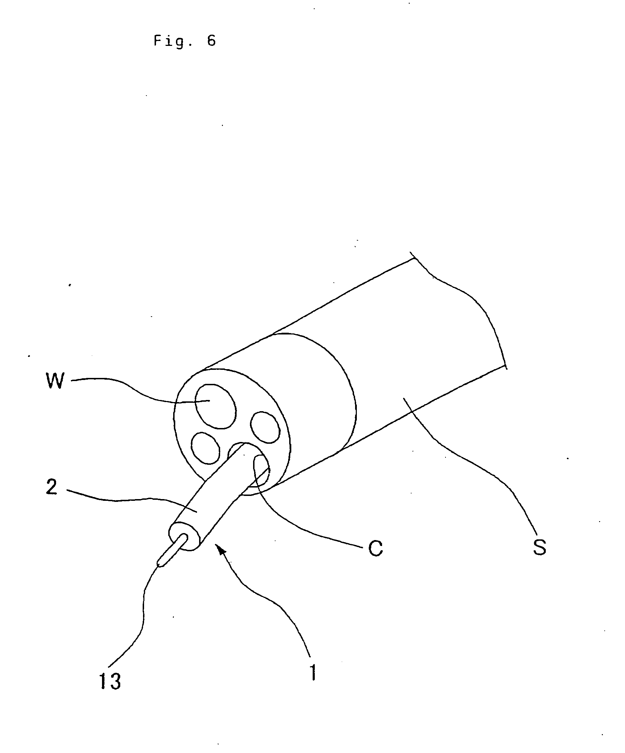

The high frequency treatment tool 1 constructed as described above is inserted in a body cavity via the treatment tool insertion channel C provided in the endoscope inserting portion S having an observing portion W as shown in Fig. 6, and used for performing treatment to exfoliate and remove a diseased mucous membrane when the diseased portion appears on the mucous membrane on the body cavity inner wall of, for example, the gullet, the stomach, the duodenum, the colon, or the like. Herein, the treatment to remove this diseased mucous membrane will be explained. This treatment is performed when the mucous membrane being diseased is found as a result of endoscopic examination.

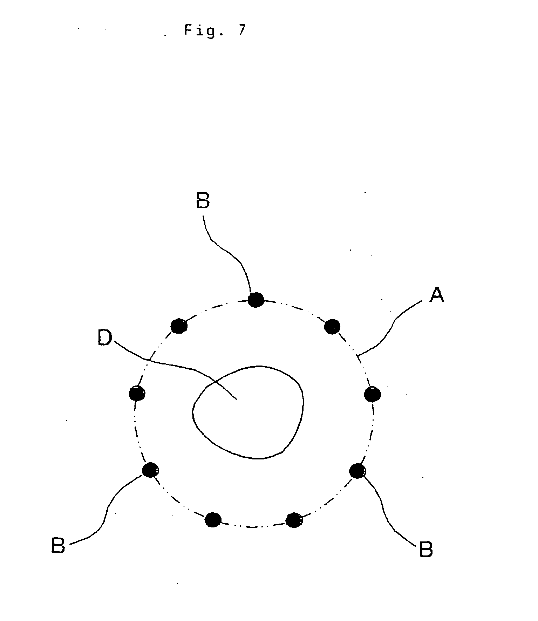

First, as shown in Fig. 7, the mucous membrane including the diseased portion D to be excised is marked so that the diseased mucous membrane region A is circled. This marking region is determined so that the diseased portion can be completely removed and damage to healthy mucous membrane is minimized. The marking can be performed by applying cauterization spots B at necessary points around the diseased mucous membrane region A, and to form the cauterization spots B, the high frequency treatment tool 1 can be used. Namely, the front end of the endoscope inserting portion S is set so as to face the outer edge of the diseased mucous membrane region A by spacing a predetermined distance, and in this state, the high frequency treatment tool 1 is inserted into the treatment tool insertion channel C and the front end thereof is made contact with the mucous membrane surface. At this time, the electrode member 13 is drawn into the insertion hole 15. No members are stuck out from the front end reference face F of this high frequency treatment tool 1, and this front end reference face F comes into surface contact with the mucous membrane surface.

In this state, the operating means 4 of the high frequency treatment tool 1 is operated to stick-out the electrode member 13 and apply a high frequency current to this electrode member 13. As a result, the portion of the mucous membrane in contact with the electrode member 13 is cauterized, whereby marking is performed. At the time of this marking, the electrode member 13 need not penetrate the mucosal layer, and the mucous membrane surface is cauterized to a degree to make it possible to recognize it from an image obtained through an observation part W of the endoscope inserting portion S. Namely, when the electrode member 13 is made contact with the mucous membrane surface, marking is formed. Of course, even when the operating means 4 is moved by a full stroke and the electrode member 13 is at the position of maximum sticking-out from the flexible sheath 2, the electrode member 13 is unlikely to come into contact with the muscle layer. The marking can be formed by using another treatment tool, and it is not necessary to employ the above-described cauterization as long as the region of the mucous membrane to be excised can be recognized through the observation part W.

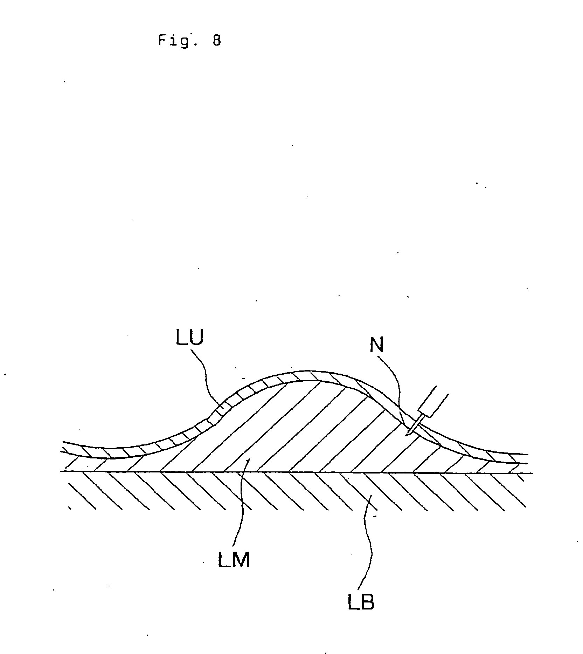

Next, normal saline solution is locally injected into the diseased mucous membrane region A as shown in Fig. 8. For this, the high frequency treatment tool 1 is temporarily extracted from the treatment tool insertion channel, and instead of this, local injecting means provided with an injection needle N on the front end of the flexible tube is inserted into the treatment tool insertion channel C. Herein, the submucosal layer LM is present between the muscle layer LB and the mucosal layer LU, and the injection needle N penetrates the mucosal layer LU and is inserted up to the submucosal layer LM to inject the normal saline solution. As a result, the submucosal layer LM is bulged and protruded. Thus, the reason for bulging the submucosal layer LM is for separating the mucosal layer LU from the muscle layer LB for smooth and safe treatment.

After sufficiently bulging the submucosal layer LM, the local injecting means is extracted from the treatment tool insertion channel C and the high frequency treatment tool 1 is inserted again. Then, the front end reference face F formed by the front end faces of the flexible sheath 2 and the stoppermember 14 of the high frequency treatment tool 1 is made contact with any portion of the outer edge of the diseased mucous membrane region A. Herein, the front end reference face F is made to correctly face the mucosal layer LU, and the front end reference face F is slightly pressed against the mucous membrane surface while the pressing force is minimized.

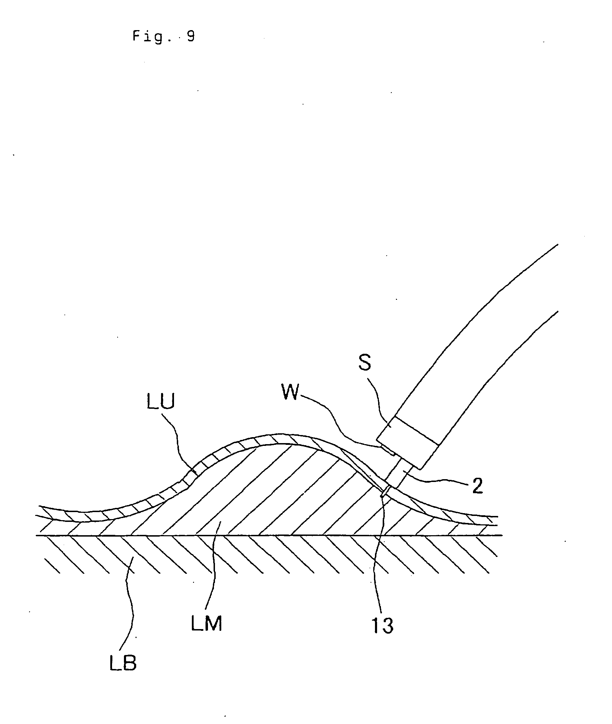

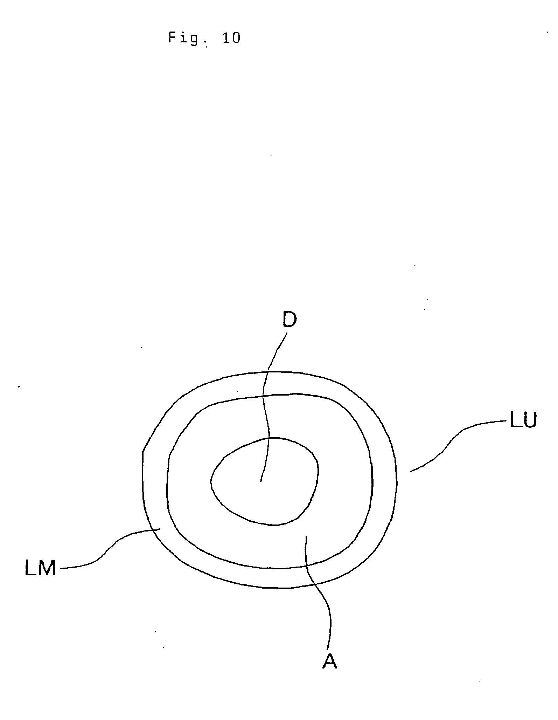

Then, the operating means 4 is operated to stick the electrode member 13 out from the front end of the stopper member 14 and supply a high frequency current to the electrode member 13 during the sticking-out operation. When the electrode member 13 sticks out most, as shown in Fig. 9, the electrode member 13 is guided up to the submucosal layer LM by penetrating the mucosal layer LU, whereby incision of the diseased mucous membrane region A is started. Then, by operations to move the endoscope inserting portion S or to bend the angle portion thereof under observation with the observation part W, the diseased mucous membrane region is incised along the cauterization spots B. Herein, the maximum sticking-out length of the electrode member 13 from the flexible sheath 2 is longer than the thickness of the mucosal layer LU and shorter than the total thickness of the mucosal layer LU and the submucosal layer LM and the submucosal layer LM is bulged by local injection, so that the mucosal layer LU can be reliably incised unless the front end reference face F extremely presses and deforms the mucous membrane surface, and the mucosal layer LU is incised without damage to the muscle layer LB. At this point, it is not especially necessary to confirm the position of the front end of the electrode member 13 through the observation part W of the endoscope inserting portion S. As a result, as shown in Fig. 10, at the outer circumference of the diseased mucous membrane region A, the mucosal layer LU is incised and the submucosal layer LM is exposed. In Fig. 10, the entirety of the diseasedmucous membrane region A is incised at a time, however, when the diseased mucous membrane region A is wide, it is desirable that a part of the region is incised and exfoliated as described later, and this operation is repeated a plurality of times.

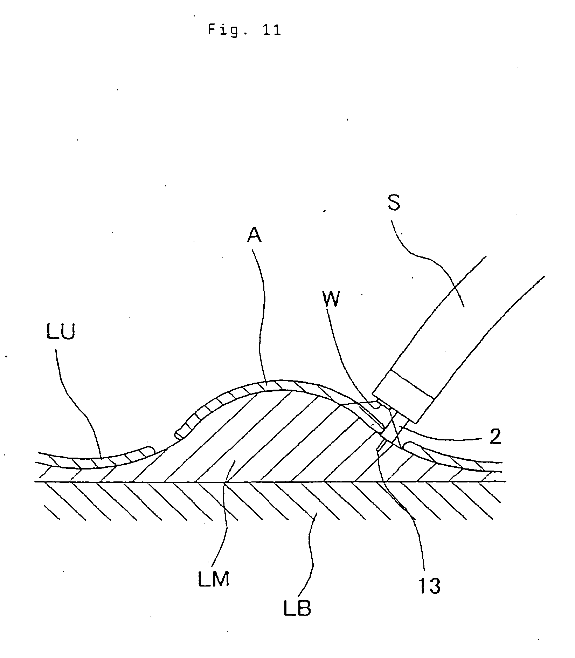

Only by incising all the circumference of the diseased mucous membrane region A, the mucosal layer LU cannot be removed. Namely, the mucous layer LU and the muscle layer LB are linked by the fibered submucosal layer LM, so that it is necessary to cut the fibers to exfoliate the layer from the muscle layer LB. This exfoliation of the mucous membrane can be performed by using the high frequency treatment tool 1. Namely, as shown in Fig. 11, the electrode member 13 sticking out from the flexible sheath 2 of the high frequency treatment tool 1 is brought to the portion of the submucosal layer LM exposed due to incision and this electrode member 13 is moved horizontally or swung, whereby the submucosal layer LM is cut. This movement can be easily performed by an operation such as bending of the front end portion of the endoscope inserting portion S. As a result, exfoliation of the mucous membrane is performed swiftly and efficiently during this exfoliation of the mucous membrane, and during the above-described incision, this treated portion may bleed. Therefore, the normal saline solution is supplied into the flexible sheath 2 by a high pressure from the connection port 3a of the connecting pipe 3. In the end face of the stopper member 14, grooves 21 communicating with the connection port 3a are opened, and the grooves 21 are not closed even when the restricting member 16 comes into contact with the stopper member 14, and no member is disposed in front of the grooves 21, so that the bleeding portion can be swiftly washed out by jetting the normal saline solution toward the bleeding portion.

When performing the exfoliation of the mucous membrane, normal saline solution needs to be replenished. The diseased mucous membrane region A has already been bulged by locally injecting the normal saline solution, however, the normal saline solution supplied may flow out or may be absorbed by the body during incision and the bulged portion may contract. Therefore, to maintain the bulged state of the submucosal layer LM, the exfoliation of the mucous membrane is performed while replenishing the normal saline solution. This replenishment of the normal saline solution is also performed through the replenishing grooves 21 provided in the outer circumference of the stopper member 14. At this time, preferably, the electrode member 13 is drawn into the insertion hole 15 of the stopper member 14, and while the front end reference face F is made contact with the submucosal layer LM, the normal saline solution is jetted into the flexible sheath 2 from the connection port 3a of the connecting pipe 3. As a result, the solution can be directly injected toward the submucosal layer LM. As a result, the submucosal layer LM to be exfoliated can be maintained in the bulged state. Thus, replenishment of the normal saline solution does not require the troublesome operation of extracting the high frequency treatment tool 1 inserted in the treatment tool insertion channel C and inserting an injection needle instead, so that the exfoliation of the mucous membrane is not interrupted. Therefore, in this point, the treatment is improved in efficiency and swiftness. In addition, no member sticks out from the front end reference face F, so that the front ends of the grooves 21 can be made contact with the submucosal layer LM, and the normal saline solution can be accurately supplied to a necessary portion. Thereby, the submucosal layer LM can be reliably maintained in a bulged state, and the exfoliation of the mucous membrane can be safely and swiftly performed with the electrode member 13.

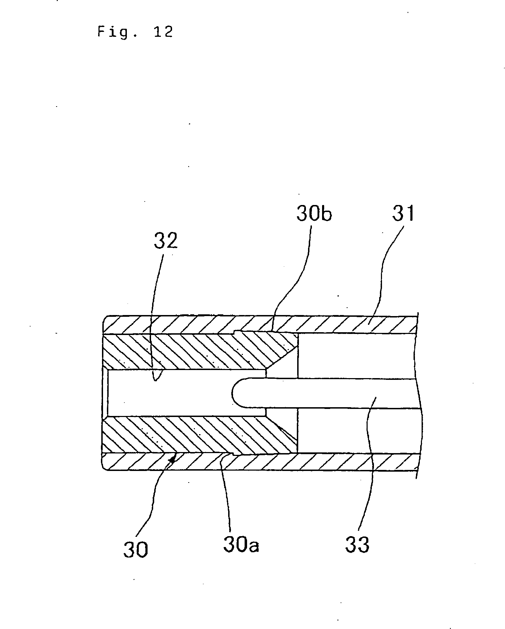

Herein, the stopper member is fixed in the front end portion of the flexible sheath so that the front end face of the flexible sheath and the front end face of the stopper member becomes the same surface. Therefore, like the stopper member 30 shown in Fig. 12, the base end side of the outer circumference is increased in diameter to form a step 30a, and a gentle slope portion 30b that reduces the diameter from this step 30a toward the base end is formed. Therefore, when the stopper 30 is attached to the flexible sheath 31, it is inserted from the base end side whose diameter is made small, the flexible sheath 31 is expanded. Then, the stopper member 30 is attached by pushing it to a position at which the front end face of the stopper member 30 almost matches with the front end face of the flexible sheath 31. Of course, by applying an adhesive with excellent heat resistance to at least one or both of the outer circumferential surface of the stopper member 30 and the inner circumferential surface of the flexible sheath 31 in advance, the stopper member 30 is fixed to the inner surface of the flexible sheath 31.

Thereby, when a force is applied in the direction of extracting the stopper member 30 from the flexible sheath 31, the stopper member 30 exerts an anchoring function due to the step 30a on the outer circumferential surface thereof biting into the inner surface of the flexible sheath 31. To more firmly fix the stopper member 30, the outer surface can be fixed to the inner circumferential surface of the flexible sheath 31 by using an adhesive, and furthermore, a screw portion is allowed to be formed on the outer circumferential surface of the stopper member 30. In the figure, the reference numeral 32 denotes the electrode member and 33 denotes the insertion hole formed in the stopper member 30.

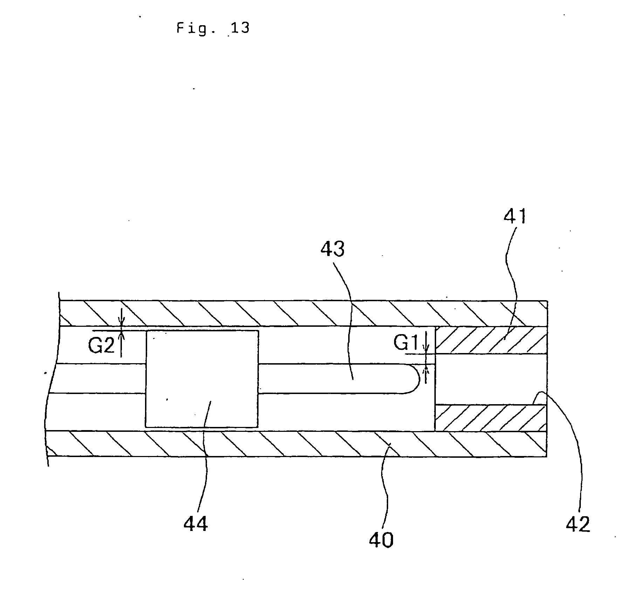

As shown in Fig. 13, by setting the diameter difference G1 between the insertion hole 42 of the stopper member 41 to be attached to the front end of the flexible sheath 40 and the electrode member 43 of the treatment tool main body to be inserted in the hole to be larger than the diameter difference G2 between the outer diameter of the restricting member 44 joined to the electrode member 43 and the inner diameter of the flexible sheath 40, center alignment between the electrode member 43 and the insertion hole 42 is performed. Therefore, even without providing the tapered portion on the base end side surface of the stopper member 41, or only by providing a slightly tapered portion, the electrode member 43 can be guided to the inside of the insertion hole 42. Herein, in the case of the construction shown in Fig. 13, to improve the center alignment with the electrode member 43, it is necessary to minimize the diameter difference between the outer diameter of the restricting member 44 and the inner diameter of the flexible sheath 40 and lengthen the length in the axial direction of the restricting member 44.

The restricting member is for restricting the sticking-out length of the electrode member in principle, and exerts a guide function for extending the electrode member straight. To restrict the sticking-out length of the electrode member, the restricting member has an outer diameter larger than the diameter of the insertion hole of the stopper member. To guide the electrode member, the length of the restricting member is necessary.

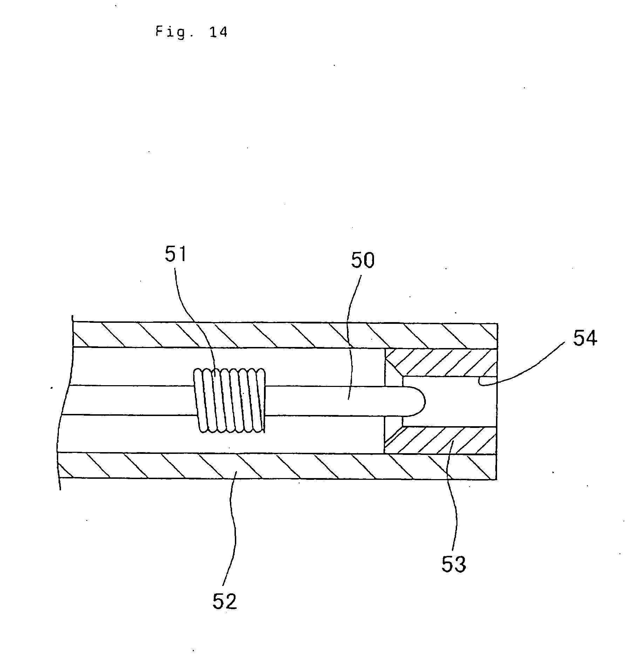

Therefore, as shown in Fig. 14, it is also allowed that the restricting member is constructed by fixing a close coil spring 51 to the outer circumferential surface of the electrode member 50, instead of the hard block shape. The outer diameter of this close coil spring 51 is set to be larger than the insertion hole 54 formed in the stopper member 53 fixed to the inner circumferential surface of the flexible sheath 52, whereby the sticking-out length of the electrode member 50 can be restricted. Thus, the close coil spring 51 does not change its outer diameter and is bendable, so that this is advantageous when the high frequency treatment tool is inserted in the treatment tool insertion channel while the angle portion of the endoscope inserting portion is bent.

By using the above-described construction, exfoliation of the mucous membrane can be smoothly, reliably, and efficiently performed.

## Claims

1. A high frequency treatment tool (1) to be inserted in a body cavity via a treatment tool insertion channel of an endoscope, comprising:
a flexible sheath (2) capable of being inserted in the treatment tool insertion channel;
a treatment tool main body comprising a flexible cord (11) and a straight electrode member (13) that can apply a high frequency current on a front end of the flexible cord, the treatment tool main body being provided inside the flexible sheath;
a stopper member (14) that comprises an electric insulating material and comprises an insertion hole (15) in which the electrode member is to be inserted;
an operating section that is connected to a base end of the flexible cord and reciprocates the electrode member between a state that the electrode member is stuck out via the insertion hole from the front end reference face and a state that the electrode member is withdrawn into the insertion hole by pushing or pulling the treatment tool main body within the flexible sheath; and
a liquid supply section that is connected to the operating section and jets a liquid from a fluid channel (21) provided at the front end reference face through an inside of the flexible sheath,
**characterised in that** the stopper member is inserted into the flexible sheath and disposes and fixes a front end position of the stopper member at the same position as a front end Face of the flexible sheath so as to form a front end reference face (F) that is adapted to press against a body cavity inner wall, the front end reference face being constituted by a front end face of the stopper member and a front end face of the flexible sheath;

2. A high frequency treatment tool (1) as claimed in claim 1, further comprising
a restricting member (16) that is provided at or near a connecting portion between the flexible cord (11) and the electrode member (13) and can come into contact with and separate from a base end of the stopper member to restrict a sticking-out length of the electrode member from the front end reference face (F).

3. A high frequency treatment tool (1) as claimed in claim 2, wherein a diameter of the insertion hole (15) formed in the stopper member is set so that the electrode member (13) is inserted therein without substantial gaps,
the fluid channel (21) is formed by one or a plurality of grooves formed on an outer circumferential surface of the stopper member (14); and
an outer diameter of the restricting member (16) is set smaller than an outer diameter of the stopper member.

## Patentansprüche

1. Hochfrequenz-Behandlungswerkzeug (1) zum Einführen in einen Körperhohlraum über einen Behandlungswerkzeug-Einführkanal eines Endoskops, umfassend:
eine flexible Hülle (2), die in der Lage ist, in den Behandlungswerkzeug-Einführkanal eingeführt zu werden;
einen Behandlungswerkzeug-Hauptkörper, umfassend ein flexibles Kabel (11) und ein gerades Elektrodenelement (13), welches einen Hochfrequenzstrom an einem vorderen Ende des flexiblen Kabels aufbringen kann, wobei der Behandlungswerkzeug-Hauptkörper im Inneren der flexiblen Hülle vorgesehen ist;
ein Anschlagelement (14), welches einen elektrischen Isolierstoff aufweist und ein Einführloch (15) besitzt, in das das Elektrodenelement einzuführen ist;
einen Betätigungsabschnitt, der mit einem Basisende des flexiblen Kabels verbunden ist und das Elektrodenelement zwischen einem Zustand, in welchem das Elektrodenelement durch das Einführloch aus der Vorderenden-Referenzfläche herausragt, und einem Zustand, in welchem das Elektrodenelement in das Einführloch zurückgezogen ist, durch Drücken oder Ziehen des Behandlungswerkzeug-Hauptkörpers innerhalb der flexiblen Hülle hin- und herbewegt; und
einen Flüssigkeitszuführabschnitt, der mit dem Betätigungsabschnitt verbunden ist und eine Flüssigkeit aus einem Fluidkanal (21), der an der Vorderenden-Referenzfläche vorgesehen ist, durch eine Innenseite der flexiblen Hülle ausstößt,
**dadurch gekennzeichnet, dass** das Anschlagelement in die flexible Hülle eingefügt ist und eine Vorderenden-Position des Anschlagelements an der gleichen Stelle wie eine Vorderenden-Fläche der flexiblen Hülle lagert und fixiert, um eine Vorderenden-Referenzfläche (F) zu bilden, die dazu ausgebildet ist, gegen eine Körperhohlraum-Innenwand zu drücken, wobei die Vorderenden-Referenzfläche gebildet wird durch eine Vorderenden-Fläche des Anschlagelements und eine Vorderenden-Fläche der flexiblen Hülle.

2. Hochfrequenz-Behandlungswerkzeug (1) nach Anspruch 1, weiterhin umfassend:
ein Beschränkungselement (16), welches an oder nahe dem Verbindungsabschnitt zwischen dem flexiblen Kabel (11) und dem Elektrodenelement (13) vorgesehen ist und mit einem Basisende des Anschlagelements in Kontakt treten oder von diesem abrückbar ist, um die Überstandslänge des Elektrodenelements gegenüber der Vorderenden-Referenzfläche (F) zu beschränken.

3. Hochfrequenz-Behandlungswerkzeug (1) nach Anspruch 2, wobei der Durchmesser des Einführlochs (15), das in dem Anschlagelement gebildet ist, derart eingestellt wird, dass das Elektrodenelement (13) im wesentlichen lückenfrei dort einführbar ist,
der Fluidkanal (21) durch eine oder mehrere Nuten gebildet ist, die an einer Außenumfangsfläche des Anschlagelements (14) ausgebildet sind, und
ein Außendurchmesser des Beschränkungselements (16) kleiner eingestellt ist als der Außendurchmesser des Anschlagelements.

## Revendications

1. Outil de traitement à haute fréquence (1) devant être inséré dans une cavité corporelle via un canal d'insertion d'outil de traitement d'un endoscope, l'outil de traitement comprenant :
une gaine flexible (2) apte à être insérée dans le canal d'insertion d'outil de traitement ;
un corps principal d'outil de traitement comprenant un cordon souple (11) et un élément formant électrode rectiligne (13) qui peut appliquer un courant à haute fréquence sur une extrémité avant du cordon souple, le corps principal d'outil de traitement étant prévu à l'intérieur de la gaine flexible ;
un élément de butée (14) qui comprend un matériau d'isolation électrique et comprend un trou d'insertion (15) dans lequel l'élément formant électrode doit être inséré ;
une section de fonctionnement qui est connectée à une extrémité de base du cordon souple et qui amène l'élément formant électrode à effectuer un mouvement de va-et-vient entre un état dans lequel l'élément formant électrode est sorti et bloqué à l'extérieur via le trou d'insertion de la face de référence d'extrémité avant et un état dans lequel l'élément formant électrode est rentré à l'intérieur du trou d'insertion en poussant ou en tirant le corps principal d'outil de traitement à l'intérieur de la gaine flexible ; et
une section de fourniture de liquide qui est connectée à la section de fonctionnement et qui projette un liquide à partir d'un canal de fourniture de fluide (21) qui est prévu au niveau de la face de référence d'extrémité avant au travers d'un intérieur de la gaine flexible,
**caractérisé en ce que** l'élément de butée est inséré dans la gaine flexible et place et fixe une position d'extrémité avant de l'élément de butée à la même position qu'une face d'extrémité avant de la gaine flexible de sorte à former une face de référence d'extrémité avant (F) qui est adaptée pour appuyer contre une paroi interne d'une cavité corporelle, la face de référence d'extrémité avant étant constituée par une face d'extrémité avant de l'élément formant butée et une face d'extrémité avant de la gaine flexible.

2. Outil de traitement à haute fréquence (1) selon la revendication 1, comprenant par ailleurs :
un élément de limitation (16) qui est prévu au niveau ou à proximité d'une section de connexion entre le cordon souple (11) et l'élément formant électrode (13) et qui peut venir en contact avec et se séparer d'une extrémité de base de l'élément de butée dans le but de limiter une longueur de sortie de l'élément formant électrode par rapport à la face de référence d'extrémité avant (F).

3. Outil de traitement à haute fréquence (1) selon la revendication 2,
dans lequel un diamètre du trou d'insertion (15) formé dans l'élément de butée est défini de telle sorte que l'élément formant électrode (13) soit inséré à l'intérieur sans écarts sensibles,
le canal de fourniture de fluide (21) est formé par une ou une pluralité de gorges formée sur une surface de circonférence extérieure de l'élément de butée (14), et
un diamètre extérieur de l'élément de limitation (16) est défini plus petit qu'un diamètre extérieur de l'élément de butée.
